# EUROPEAN PATENT APPLICATION

(11) **EP 2 169 577 A1**
(43) Date of publication of application: **31.03.2010**
(21) Application number: 09012155.9
(22) Date of filing: 24.09.2009
(51) Int. Cl.: G06F 19/00

(54) **Method and system for medical imaging reporting**

(30) Priority: 25.09.2008 US 136695 P
(71) Applicant: Algotec Systems Ltd., 43107 Raanana (IL)
(72) Inventor: Schreiber, Reuven, 34366 Haifa (IL); Aradi, Yinon, 69983 Tel-Aviv (IL); Dorman, Yonatan, 47128 Ramat-HaSharon (IL); Benjamin, Menashe, 43555 RaAnana (IL)
(74) Representative: Kurig, Thomas

(57) **Abstract**

A method for supporting a preparation of medical report for a patient. The method comprises acquiring one or more medical imaging studies and/or medical records which are related to the patient, automatically matching a report template to the one or more medical imaging studies and/or medical records according to at least one characteristic thereof, presenting the matched report template to allow a user to provide a diagnosis of the one or more medical imaging studies and/or medical records, and embedding the diagnosis in the matched report template.

## Description

### FIELD AND BACKGROUND OF THE INVENTION

The present invention, in some embodiments thereof, relates to a system and a method for creating a medical report and, more particularly, but not exclusively, to a system and a method for creating a medical report based on one or more radiology studies.

The technology for acquiring and processing medical record and images has advanced dramatically in recent years. Radiology information systems (RIS) and picture archiving and communication systems (PACS) have become more efficient; adopted more logical, effective, and consistent user interfaces; and taken advantage of Web technology, the proliferation of broadband, and faster and better hardware to efficiently distribute images outside the confines of radiology departments.

In most hospitals and radiology centers, the medical imaging studies are transferred to a picture archiving communication system (PACS) before being accessed by the radiologists. The PACS is installed on one or more of computers, which are dedicated for storing, retrieving, distributing and presenting the stored 3D medical imaging studies. The medical imaging studies are stored in an independent format. The most common format for image storage is digital imaging and communications in medicine (DICOM). Usually, the PACS stores medical imaging studies on servers that provide central storage and access to the images. The servers are connected to the PACS network and allow radiologists and other healthcare staff which are connected thereto via client terminals which are connected to the PACS network in Clinics, hospitals and/or home settings to access the medical imaging studies.

PACS are often implemented on network systems, such as local area networks (LANs). Such a PACS includes a server system for controlling the transfer of medical imaging study data from DICOM modalities to multiple client terminals of the LAN. Since medical imaging study files are typically large data files, it is not uncommon for simultaneous data requests from multiple client systems to heavily burden the existing bandwidth of the network's data link.

An effort to link between the medical image and medical record from clinical information systems and other databases has been made to allow a user to seamlessly access them from a single point of end-user interaction.

For example, US Patent Publication Application No. 2003/0187689 published on October 2, 2003, describes a method and an apparatus for providing fully integrated information processing, management and communication functions in a fully integrated RIS-PACS system for a radiology department/healthcare environment by employing brokerless interface methods to synchronize patient and exam data entities in a RIS database and a PACS database within the RIS-PACS system and to generate direct database calls to the RIS and PACS databases. The running of RIS and PACS applications within the RIS-PACS system is initiated from client workstations over a web interface. Information from the databases is accessed by the client workstations. Also, the system may communicate between internal PACS applications and an external RIS system over a HL7-based interface or between internal RIS applications and an external PACS system over a standard medical communications interface such as HL7 and DICOM.

The integration of medical images and medical record allows the generation of a report creation system that provides access from either a RIS or a PACS user interface. A user may initiate a medical reporting session via the RIS and/or the PACS user interface. This action electronically transfers patient and exam information to the report creation system and provides uninterrupted workflow for the radiologist. An end user, such as a referring physician, is able to review completed reports in a hospital information system (HIS)/electronic patient record (EPR) application and access images by launching the PACS viewer from within the EPR user interface, for example by allowing the user to click on a standard hyperlink, which launches the PACS viewing application and notifies the PACS application, by reference to the unique study identifier in the tag, to retrieve the applicable images.

In addition, different reporting systems have been developed to allow user, such as a radiologist, to display medical records. For example, US Patent Publication Application No. 2003/0187689 published on February 16, 2006 discloses a computer program product for analysis of a source medical image data set in a medical imaging system 211, being operable to: identify a user identity; identify at least one role linked to the user identity; load a source medical image data set; allow the user to review the loaded source medical image data set; allow the user to, based on role privileges, interactively perform processing operations on the loaded source medical image data set; create a processing protocol; allow the user to interactively specify at least one medical image data set characteristic to be associated with a processing protocol associater; allow the user to interactively restrict which user(s) to be associated with a user access right of the processing protocol by specifying which role(s) or which user identity/identities to be associated with the user access right; allow the user to interactively further restrict the role privileges; store the processing protocol in a central unit, and, if the user is associated with a user access right of a stored processing protocol, reload the stored processing protocol; allow the user to interactively choose the processing protocol for application, and apply the stored processing protocol.

### SUMMARY OF THE INVENTION

According to an aspect of some embodiments of the present invention there is provided a method for supporting a preparation of medical report for a patient. The method comprises acquiring at least one of a medical imaging study and a medical record related to the patient, automatically matching a report template to the at least one of the medical imaging study and the medical record according to at least one characteristic thereof, presenting the matched report template to allow a user to provide a diagnosis of the at least one of the medical imaging study and the medical record, and embedding the diagnosis in the matched report template.

Optionally, the automatically matching comprises automatically matching the report template according to a combination of the medical imaging study and the medical record.

Optionally, the allowing comprises dynamically adjusting the report according to the diagnosis.

Optionally, the at least one characteristic comprises a member of a group consisting of: a modality type, and anatomic site, a related patient medical history, a related patient radiation exposure, a related patient sex, a related patient age, and a related patient pathology.

Optionally, the matching comprises analyzing the at least one medical imaging study to identify an anatomic site in the body of the patient and matching the report according to the anatomic site.

Optionally, the medical record being acquired from a member of a group consisting of: an electronic patient record (EPR) system, a hospital information system (HIS), a radiology information system (RIS), clinical information system (CIS) record, and a database hosting medical records related to a plurality of patients.

Optionally, the embedding comprises adding data from the at least one medical imaging study to the report.

Optionally, the report template is matched from a plurality of report templates, the acquiring comprising acquiring statistical data related to at least one previously selected report, the matching being performed according to the statistical data.

Optionally, the method further comprises documenting the matching, wherein the documenting allows adjusting at least one prospective report, the at least one prospective report being filled in by the user.

Optionally, the method further comprises automatically updating the report with related data from the at least one of the medical imaging study and the medical record.

Optionally, the acquiring comprises acquiring a plurality of medical imaging studies related to the patient, each the medical imaging study being received from another medical imaging study database.

Optionally, the embedding comprises automatically identifying at least one miscorrelation in the at least one of the medical imaging study and the medical record and adding a notice pertaining to the at least one anomaly to at least one of the report, the medical imaging study, and the medical record.

More optionally, the identifying comprises estimating a risk factor to the patient according to the at least one of the medical imaging study and the medical record and adjusting the notice according to the risk factor.

Optionally, the embedding comprises estimating the amount of radiation the patient received according to at least one of the at least one medical imaging study and the medical record and generating an alarm according to the estimating.

Optionally, the embedding comprises automatically identifying at least one anomaly in the at least one of the medical imaging study and the medical record, further comprising presenting the at least one anomaly to the user.

Optionally, the allowing comprises recording a dictation of the diagnosis, converting the recording to a text segment, and adding the text segment to the report.

Optionally, the acquiring extracting billing information from the at least one of the medical imaging study and the medical record, the matching being performed according to the billing information.

Optionally, further comprising confirming a coherency between the diagnosis and data extracted from the at least one of the medical imaging study and the medical record.

More optionally, the method further comprises alarming the user according to the confirming.

More optionally, the diagnosis comprises a diagnosis related to a certain anatomic site of the at least one medical imaging study, the coherency comprises a match between certain anatomic site and the diagnosis.

More optionally, the method further comprises using a computer aid diagnosis (CAD) for processing the at least one medical imaging study and updating the report according to the processing.

Optionally, the matching comprises matching a radiology analysis standard according to the at least one of the medical imaging study and the medical record and analyzing the diagnosis to verify a compliance of the user with the matched radiology analysis standard.

Optionally, further comprising segmenting at least one anatomic site in the at least one medical imaging study and adjusting the report according to the segmenting.

Optionally, the method further comprises further comprising verifying the consistency of the report.

Optionally, the diagnosis comprises a member of a group consisting of: a suspected pathology, a body part, a point of view, a level of certainty, a diagnosis, a recommended treatment, and a patient condition.

Optionally, the method further comprises comprising identifying a match between at least one of the diagnosis and the at least one medical imaging study and at least one of a plurality of exemplary medical imaging study reports, and adding the at least one exemplary medical imaging study report to the report.

Optionally, the acquiring comprises receiving a diagnosis indicator from the user, the automatically matching being performed according to the diagnosis indicator.

Optionally, the matching comprises a member of a group consisting of: selecting the report template from a plurality of report templates, adjusting the report template, and merging a plurality of report subsection templates for creating the report template.

Optionally, the presenting comprises selecting a presentation protocol according to the at least one of the medical imaging study and performing the presenting according to the presentation protocol.

According to an aspect of some embodiments of the present invention there is provided a client terminal for preparing a medical report. The client terminal comprises an acquiring module configured for acquiring at least one of a medical record and a medical imaging study related to a patient, a report generation module configured for generating a report by matching a report template of a plurality of report templates to the at least one of the medical record and the medical imaging study, and a man machine interface (MMI) configured for allowing a user to provide a diagnosis about the at least one of the medical record and the medical imaging study. The diagnosis is embedded into the report template to form the medical report.

Optionally, the report generation module is configured for dynamically adjusting the report according to the diagnosis.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user's diagnosis input device such as a keyboard or mouse are optionally provided as well.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flowchart of a medical reporting session for preparing a radiology report pertaining to a patient, according to some embodiments of the present invention;
FIG. 2 is a schematic illustration of a client terminal for preparing a radiology report which is connected to one or more medical imaging systems and electronic patient record systems, according to some embodiments of the present invention;
FIG. 3 is a flowchart of an exemplary process for matching a report template to one or more medical studies and/or records, according to some embodiments of the present invention;
FIG. 4 is a flowchart of an exemplary process for using the received data for constructing a report, according to some embodiments of the present invention;
FIGs. 5A and 5B are screenshots of an exemplary user interface for presenting an exemplary report, according to some embodiments of the present invention;
FIGS. 5C-5G are members of a set of screenshots that illustrates the process of a report generating, for example according to the method depicted in FIG. 1 and described above;
FIGS. 5H-5I are members of a set of screenshots that illustrates the process of a teach report file generating, according to some embodiments of the present invention;
FIG. 6 is a flowchart of a diagnosis process, according to some embodiments of the present invention;
FIG. 7 is a flowchart of a method for preparing a radiology report pertaining to a patient, according to some embodiments of the present invention; and
FIG. 8 is a schematic illustration of a platform for managing medical data records which are stored in multiple local medical imaging systems and for allowing a client terminal to prepare a radiology report on the basis of imaging studies from various systems, according to some embodiments of the present invention.

### DESCRIPTION OF SPECIFIC EMBODIMENTS OF THE INVENTION

The present invention, in some embodiments thereof, relates to a system and a method for creating a medical report and, more particularly, but not exclusively, to a system and a method for creating a medical report based on one or more radiology studies or sequences.

According to some embodiments of the present invention there are provided methods and systems for enhancing a medical reporting session, such as a session in which a radiology report is created, by extracting data from one or more medical studies and/or medical records which are related to a patient and embedding the extracted data in a diagnostic report that is related thereto. In such embodiments, the reports may include segments of data and/or images which are related to the patient and may be used for clarifying the findings of the reports and/or for reducing common human errors which may be made by the radiologist, which may be referred to herein as the user, that diagnoses the patient. The extracted data may be used for matching medical data from an external source to the report. The matched data may be embedded into the report and/or linked thereto.

Optionally, the reports are adjusted according to the diagnosis that is provided by the user. The adjusting may be performed while and/or after the user provides her diagnosis, for example during a diagnosis dictation.

Optionally, the extracted data is presented to the radiologist according to a presentation protocol that has been adjusted for the diagnosis of the received one or more medical studies and/or medical records. The presentation protocol enhances the diagnosis process and reduces the common human errors which may be made by the radiologist. As further described below, the presentation protocol may reduce general reporting errors, such as reporting on the wrong study and/or the wrong patient.

According to some embodiments of the present invention there is provided a method and a client terminal, such as a personal computer, for supporting a preparation of medical report for a patient. The method is based on acquiring one or more medical imaging studies, such as DICOM objects and/or medical records, such as RIS objects, which are related to the patient and using them for automatically matching a report template. Optionally, one or more characteristics, such as pathology, anatomic site, and a modality, are extracted from the one or more medical imaging studies and/or records and matched with one or more tags of report templates which are stored in a designated repository. Such a matching allows the identification of a matching template that enhances that medical reporting session, for example by determining the manner in which views of the medical imaging study are presented. Now, the matched report template is used for generating a report. The generation of such a report allows a user, such as a radiologist, to fill in her diagnosis on the basis of the medical imaging studies and/or record.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

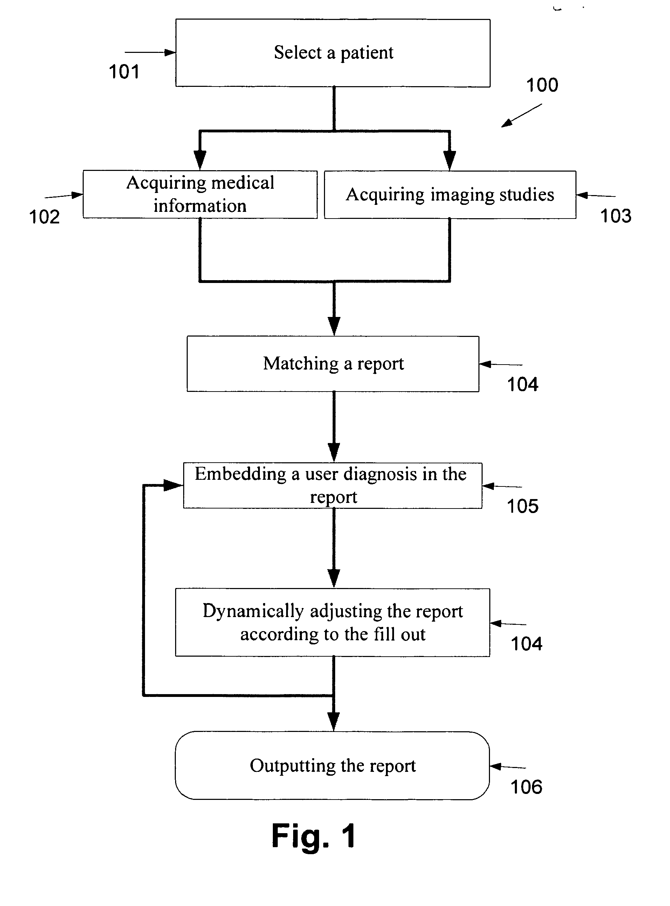

Reference is now made to FIG. 1, which is a flowchart 100 of a medical reporting session for preparing a radiology report pertaining to a patient, according to some embodiments of the present invention. As commonly known, radiology reports are the primary means of communication between a radiologist and a referring physician and a mean for documenting the medical condition of the patient and the conclusion regarding the medical condition. The reports reflect the attitude, perception and capability of the radiologist and serves as a legal document. As shown at 100, the generated report is based on data that is acquired from one or more medical imaging studies and/or medical records which are related to the patient. As used herein, a medical imaging study means a two dimensional (2D) medical imaging study a three dimensional (3D) medical imaging study, a four dimensional (4D) medical imaging study, a spatial image, a sequence of CT scan images, a sequence of MRI scan images, a sequence of PET-CT scan images, a Single photon emission computed tomography (SPECT) image, a medical imaging study with additional information layers, a DICOM object and/or any output of a medical imaging modality.

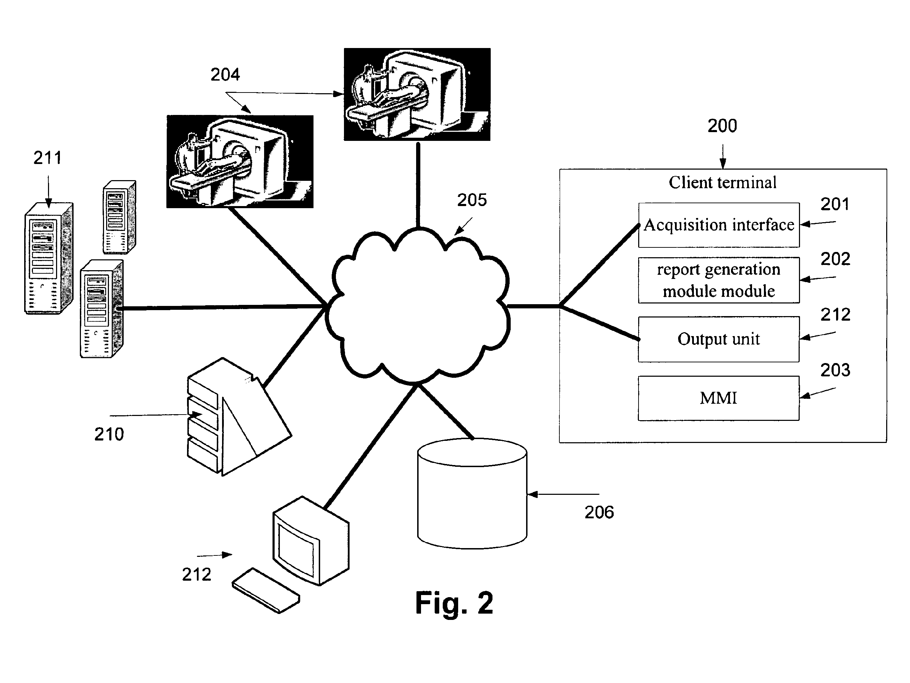

Reference is also made to FIG. 2 is a schematic illustration of a client terminal 200 for preparing a radiology report that is connected to one or more medical imaging systems 211 and electronic patient record systems 210, according to some embodiments of the present invention. The client terminal 200 prepares the radiology report according to one or more medical records and/or medical imaging studies which are related to a patient, and received from the aforementioned systems. As used herein, a client terminal means a personal computer, a laptop, a thin client, a Smartphone, and a personal digital assistant (PDA). The client terminal 200 is connected to a communication network 205, such as the internet.

The client terminal 200 is designed to acquire, optionally, via the communication network 205, the medical imaging studies from one or more medical imaging systems 211. Optionally, each medical imaging system 211 is dedicated to the storage, retrieval, distribution and presentation of medical images, such as the PACS. In such embodiments, each local medical imaging system 211 consists of a central server, such as a DICOM server, that stores a plurality of medical imaging studies, such as outcomes of medical imaging, for example medical imaging studies, and connected to one or more other client terminals 212. Alternatively or additionally the client terminal 200 may acquire the medical imaging studies directly from a modality 204 that has been used for capturing it. For example, CT images may be acquired directly from the CT modality and MRI images may be acquired directly from the MRI modality.

The client terminal 200 is designed to acquire, optionally, via the communication network 205, the one or more medical records from the one or more electronic patient record (EPR) systems 210, which may be radiology information systems (RISs) or the hospital information systems (HISs). Optionally, each EPR system 210 stores, manipulates and/or distributes one or more medical records of each one of a group of patients. As used herein, a medical record means a RIS record, a HIS record, a clinical information system (CIS) record and/or any other record that includes medical record that is related to a certain patient.

In order to allow a user, such as a clinician, a referring physician, and/or a radiologist to acquire data from both the EPR system 210 and the one or more medical imaging systems 211, a network-based infrastructure that integrates data from the one or more medical imaging systems 211 and the one or more EPR systems 210 is provided. Optionally, the client terminal 200 is connected to a system that supports a desktop integration of RIS and PACS functions, for example as described in Patent Application No. 61/071,709 and Patent application No. 61/071,708 co-filed on May 14, 2008, which are incorporated herein by reference.

As described above, the method 100 and the client terminal 200 are designed to allow a user to prepare a report that is related to a certain patient. Optionally, a plurality of client terminals are connected to the same EPR and medical imaging systems 211 202, 201.

First, as shown at 101, the user selects a patient. Optionally, each client terminal 100 may display a graphical user interface (GUI) that allows the user to select the patient that she wants to prepare the report on. Optionally, the GUI may allow the user to search the patient in the one or more EPR systems 210 and/or the medical imaging systems 211, for example using a search engine and/or an access to a list that contains all the available patients. Additionally or alternatively, the user selects the patient from a work list that contains the patients that have been assigned to her. As depicted in FIG. 2, the client terminal 200 comprises a man machine interface (MMI) 203 that allows the user to make the selection. Optionally, the MMI includes a display, such as a liquid crystal display (LCD) and an input device, such as a keyboard and a mouse that allows the user to make the selections.

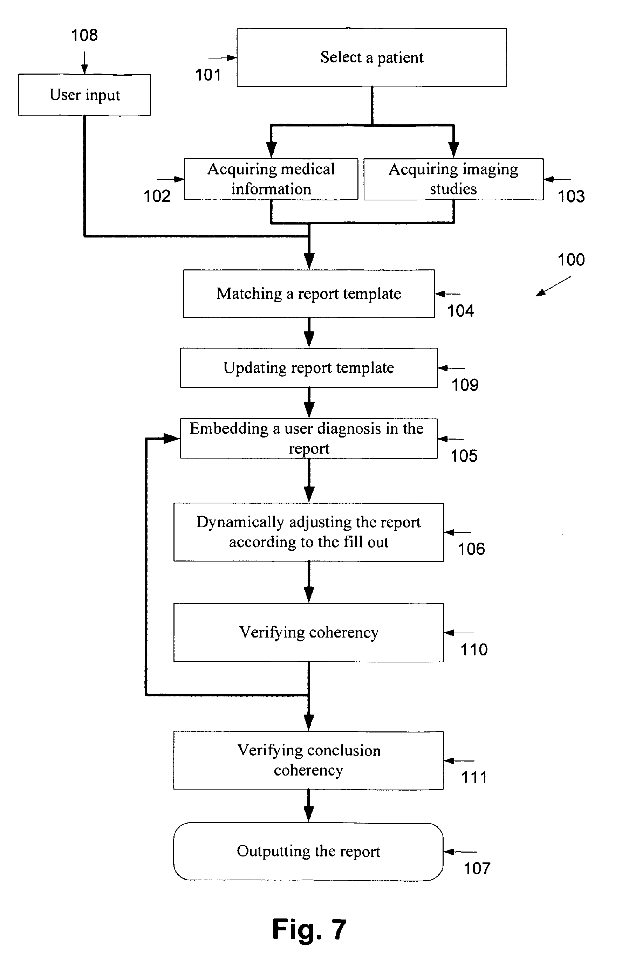

As shown at numeral 108 of FIG. 7, the user may enter additional information that is related to the medical reporting session. Optionally, the user may define the diagnosis that she is about to perform. Such inputs may be used for selecting which one or more medical imaging studies and/or medical records to retrieve from the storage devices 210, 211. For instance, if the medical imaging system 211 a plurality of medical imaging studies which are related to the patient, for example imaging studies which are related to different anatomic sites and/or modalities, the user inputs may be the basis for selecting the medical imaging study that is requested by the user of the client terminal.

Then, as shown at 102 and 103, the one or more medical records and/or medical imaging studies which are related to the patient are acquired. Optionally, the one or more medical imaging studies are DICOM objects acquired from a PACS system and one or more of the medical records are RIS records acquired from RIS systems. As depicted in FIG. 2, the client terminal comprises an acquisition interface 201 for acquiring the one or more medical imaging studies and/or medical records which are related to the patient. Optionally, as shown at FIG. 2, the acquisition interface 201 is connected to the communication network 205.

Now, as shown at 104, a report template for the report is matched according to the one or more medical imaging studies and/or medical records. The matching is based on selecting the report template from a plurality of report templates which are stored in a repository, adjusting the report template, and/or merging a plurality of report subsection templates for creating the report template, for example as described below. Optionally, the client terminal comprises a report generation module 202 that is designed to acquire and/or to adjust a report template from a repository of templates. Optionally, the report generation module 202 acquires and/or adjusts one or more sections of a report template according to the one or more medical imaging studies and/or medical records.

Optionally, the report generation module 202 accesses a template dictionary that hosts a plurality of report templates and selects a report template according to the one or more medical imaging studies and/or medical records which are related to the patient. The template dictionary may be locally stored in the client terminal 200 or in an independent database, which is optionally connected to the communication network 205, as shown at 206. Optionally, each report template comprises a plurality of editable and/or removable sections. Each section is optionally tagged with a section identifier, such as a name tag, and/or a section description that describes the data that should be filled in the respective section. Optionally, the template dictionary stores report subsection templates, each related to one or more characteristics of the medical imaging studies and/or medical records. In use the report generation module 202 identifies a match between the report subsection templates and the acquired medical data and merges them to create an appropriate report template.

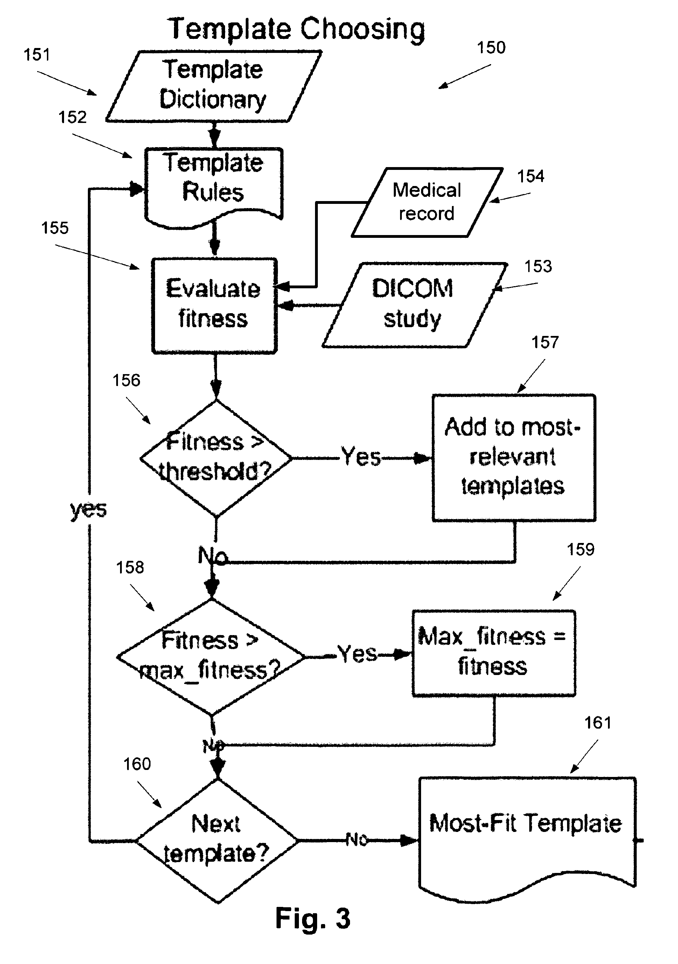

Reference is now also made to FIG. 3, which is a flowchart 150 of an exemplary process for matching a report template, according to some embodiments of the present invention. As used herein matching a report template means matching, adjusting, selecting, arranging, and/or manipulating a report or a report template.

FIG. 3 depicts the process in which the suitability of each report template is evaluated according to the one or more received medical records and/or studies. First, as shown at 151, the report generation module 202 accesses the template dictionary. Optionally, each template is tagged with a set of template rules that define the medical imaging studies and/or studies to which it suitable. For example to which modality, anatomic site, patient type, pathology, and/or combination thereof the template is suitable. As shown at 152, for each template, the report generation module 202 accesses the set of rules. As shown at 153 and 154, the report generation module 202 extracts respective data from the one or more received medical records and/or studies and evaluate. Then, as shown at 155, the report generation module 202 matches between the extracted data and the set of rules in order to evaluate the fitness and/or suitability of the template to the received data.

Optionally, the extracted data includes the modality which has been used for capturing the related one or more medical imaging studies. As used herein, a modality means angioscopy-retired, biomagnetic imaging, color flow Doppler-retired, cinefluorography, colposcopy retired, computed radiography, cystoscopyretired, computed tomography, duplex Doppler retired, digital fluoroscopy, diaphanography, digital microscopy, digital subtraction angiography retired, digital x-ray, echocardiography, endoscopy, Fluorescein angiography, fundoscopy, hard copy, laparoscopy retired, laser surface scan, magnetic resonance angiography retired, mammography, magnetic resonance, magnetic resonance spectroscopy retired, nuclear medicine, PET, radio fluoroscopy, radiographic imaging with a conventional film screen, dose radiotherapy dose, image radiotherapy image, plan radiotherapy plan, struct radiotherapy structure set, structured reporting, single-photon emission computed tomography retired, thermography, ultrasound, videofluorography, x-ray angiography, external camera, and electrocardiograms. In such an embodiment, the template dictionary includes different templates for different modalities, different anatomic sites, and/or different combinations modalities and/or anatomic sites. Optionally, each template includes a basis for possible findings that are appropriate for the given modality and/or anatomic site and the report generation module 202 evaluates the report templates according to the anatomical site that is depicted in the medical imaging study and/or the modality which has been used for capturing it.

Optionally, in order to determine the related anatomical site and/or modality the report generation module 202 analyzes the medical imaging study. The analysis may be performed by a process of text analysis of the fields of the medical imaging study. For example, if the medical imaging study is a DICOM object, it is usually defined according to the DICOM standard, which is incorporated herein by reference. In such an embodiment, the DICOM object includes a modality field that defines the modality which has been used for capturing the medical imaging study that is stored therein. For example, is the DICOM object includes data acquired by a modality of type biomagnetic imaging, the modality field denotes BI and if the DICOM object includes data acquired by a modality of type endoscopy, the modality field denotes ES.

Optionally, the analysis may be performed by an image processing of the medical imaging study. In such an embodiment, the medical imaging study may be analyzed to identify the depicted anatomical site and/or the modality which has been used for capturing it. Such an image processing may be performed by many frequently used and/or commercially available techniques of segmenting organs, optionally using anatomical models of organs. For example, the segmentation may be performed by a region growing approach where respectively adjacent voxels are analyzed starting from seed points, which may be prescribed by the user and identified as part of the vessel structure upon fulfillment of specific conditions. As one condition for the membership of an anatomical structure, it is possible, for example, to check whether the voxel falls into a prescribed Hounsfield units (HU) range. Optionally, the segmentation is supported by an anatomical model that is based on anatomical knowledge including expected volume, shape, relative position, and respective radiation attenuation of organs that provides feature constraints that guide the segmentation process.

Now, as shown at 156 a fitness score is given to the suitability of the report template to the received data. If the fitness score is above a predefined threshold, the report template is tagged as the most relevant template, as shown at 157. Else, as shown at 158, the fitness score is matched with the highest score which has been given during the exemplary process for matching a report template. For brevity, this highest score is marked as "Max_fitness" score. As, shown at 159, if the fitness score of the report template is higher than the Max_fitness, the Max_fitness is updated according to the fitness score of the report template and the report template is tagged as the most relevant template. Else, as shown at 160, the next template is probed according to aforementioned 151-159. If no more templates are available, as shown at 161, the most relevant template is selected and the exemplary process for matching a report template is ended.

As shown at numeral 109 of FIG. 7, after the related anatomical site and/or modality have been identified and a matching template have been acquired, as shown at 104, the report generation module 202 automatically fills out and/or adjusts the sections thereof according to the medical records of the related patient. Additionally or alternatively, the report generation module 202 automatically adds the received medical imaging study or images therefrom to the report.

Usually, each medical imaging study is attached with radiation related data such as a contrast material, a CT dose index (CTDI), a dose length product (DLP), the number of exposures, such as posterior-anterior (PA) view and/or lateral view in chest X-ray. Optionally, the radiation related data is embedded into the report, as shown at 109. Optionally, the radiation related data is used for generating an alarm, for example as described below.

Optionally, the report generation module 202 automatically fills out and/or adjusts the sections of the report template according to information from external radiology databases. Optionally, data that is extracted from the one or more received medical records and/or studies, is used for searching relevant articles and/or exemplary medical records and/or studies, for instance using search engines, such as search engines of databases of medical images, such as http://www.yottalook.com, which the content thereof is incorporated herein by reference. Optionally, the relevant articles and/or exemplary medical records and/or studies or links thereto are embedded, automatically and/or semi automatically, into the report. Optionally, the relevant articles and/or exemplary medical records and/or studies are used for adjusting and/or processing the one or more received medical records and/or studies. Optionally, the report generation module 202 is designed to add automatically and/or semi automatically references to relevant articles and/or exemplary medical records and/or studies according to the finding section of the report.

Optionally, the report generation module 202 is designed to update, automatically and/or semi automatically, databases of medical images with the reports and/or segments thereof.

Optionally, the report generation module 202 automatically and/or semi automatically detects miscorrelation between the one or more acquired medical studies and the acquired medical information. In such an embodiment, the report generation module 202 may generate a notice, such as an alarm to notify the user about the detected miscorrelation, for example as described below. Additionally or alternatively, a notice may be added to the report.

Optionally, the report generation module 202 automatically and/or semi automatically detects an anomaly in the one or more acquired medical studies and/or in the acquired medical information. The anomaly may be detected using a CAD system, as commonly known in the art, and/or by matching between the one or more acquired medical studies and/or in the acquired medical information and exemplary templates and/or images of respective healthy anatomical sites. In such an embodiment, the report generation module 202 may generate a notice, such as an alarm to notify the user about the detected miscorrelation, for example as described below. Additionally or alternatively, a notice may be added to the report.

As described above and shown at 102, one or more medical records which are related to the patient are acquired. The medical records are optionally acquired via the communication network 205, for example from a medical record database 210 of a hospital, a healthcare institute, and/or any other database that hosts medical record about patients. Each medical record is optionally received as a core data set of relevant administrative, demographic, and clinical information facts about the patient and the patient's healthcare, covering one or more healthcare encounters, see ASTM standards, such as ASTM E2369-05, E1382, E1384, E1762, E1869, E1985, E1986, E2084, E2085, E2086, E2147, E2182, E2183, E2184, E2211, and E2212, which are incorporated herein by reference. For example, the medical record may include identifying information about the patient, such as ID number, name, age, sex, a summary of the patient's health status, such as pathologies, medications, and allergies, and basic information about insurance, advance directives, care documentation, and the patients care plan.

The filling in and/or adjusting of the report template according to the medical record is optionally performed by matching between the identifiers and/or descriptions of the sections of the report template and data that is extracted from the medical record.

Additionally or alternatively, the report template may be selected and/or adjusted according to the information that is provided in the medical records. Optionally, the template dictionary hosts report templates which are designated for different pathologies and/or clinical stages. Optionally, the template is selected and/or adjusted according to the user's preferences in similar cases based on his past usage.

Optionally, the template is selected and/or adjusted according to billing information that is related to the patient. The billing information may be acquired from a billing server and/or from one of the storage devices 210, 211. Such billing information may be extracted from codes of clinical indications, such as the International Classification of Diseases (ICD-9) codes and radiographic techniques, such as the current procedural terminology (CPT). In such an embodiment, text analysis may be used for detecting medical data that is needed in order to select the report template.

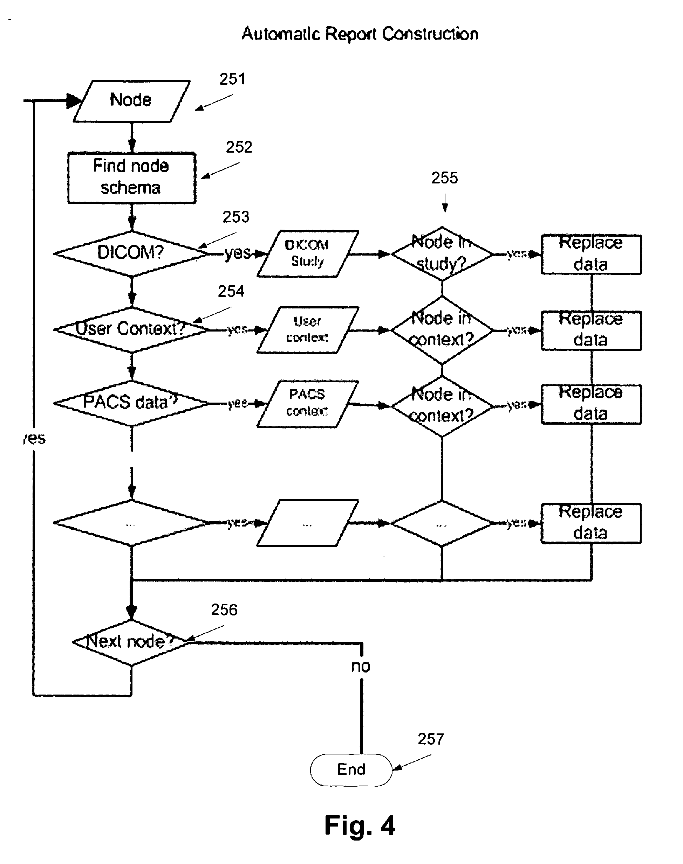

Reference is now also made to FIG. 4, which is a flowchart of an exemplary process for using the received data for constructing a report, according to some embodiments of the present invention. For brevity, each data element from the one or more medical imaging studies and/or medical records which have been acquired as shown at 102 and 103, may be referred to herein as a node. For each node, as shown at 251 a data schema is found, as shown at 252. For example, if the node is a DICOM object schema 253 is selected and of the node is medical information of a user, such as a RIS object, schema 254 is selected. As shown at column 255 after a node has been identified, other nodes which are related thereto are searched. The identification of related node allows using a combined template that fills in data and/or being adjusted according to data that is extracted from a number of nodes and/or from a deduction that is based on data that is extracted from a number of nodes. As shown at 256, the process may be repetitive. As shown at 257, this process ends after all the nodes have been checked and/or incorporated into the report.

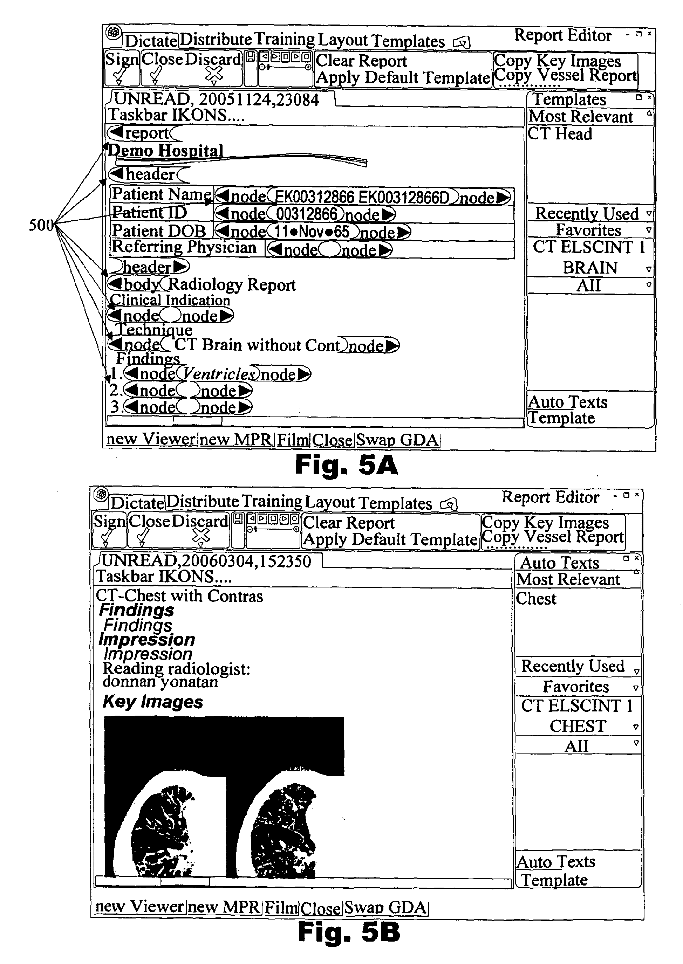

Now, as shown at 105 the client terminal 200 embeds a user diagnosis in the report. The client terminal 200 allows the user to diagnose the one or more acquired medical imaging studies and to fill out the report accordingly. Optionally, the one or more medical imaging studies, the one or more medical records, and the report template, which is optionally at least partly automatically filled as described above, are presented to the user, for example using the MMI 203. For example, FIG. 5A depicts a screenshot of an exemplary report with sections 500 which may be filled automatically, as described above and below. The automatic filling in of these sections reduces the time it takes the user to prepare the report and improve its preparedness and consistency, as further described above and below.

Optionally, the one or more medical imaging studies and/or medical records or any portion thereof are presented to the user according to a presentation protocol that has been adjusted for the diagnosis of the received one or more medical studies and/or medical records. The report generation module 202 selects the matching presentation protocol from a repository that hosts a plurality of tagged presentation protocol, optionally in a similar manner to matching a report template, for example as described above. The presentation protocol enhances the diagnosis process and reduces the common human errors which may be made by the radiologist. The presentation protocol may determine the order in which views of a medical study are presented and/or arranged, the size in which a medical study is presented, which medical imaging studies and/or medical records are presented, the order in which the medical imaging studies and/or medical records are presented and the like.

Optionally, the report generation module 202 displays and/or mark the medical imaging study that is related to the report and/or the one or more anatomic sites in the medical imaging study which are related to the report. Such a marking or selective presentation of anatomic sites may be used for reducing common human errors, such as swapping one side of an organ with another, swapping one limb with another, swapping the anterior portion of an organ with the posterior portion thereof, swapping the superior portion of an organ with the inferior portion thereof, and naming wrong anatomy in an organ, for example humerus as a leg. For example, if the report a diagnosis of a right side kidney and the medical imaging study includes imaging of both the right side and the left side kidney, only the right side of the kidney is presented to the user or marked and any side swapping of the kidneys is avoided. As used herein, marking means coloring, segmenting, highlighting and/or adding a virtual contour.

The MMI 203 optionally allows the user to fill out sections of the reports by means of typing, selecting, and/or dictating. The MMI 203 optionally comprises a microphone that allows the user to dictate his diagnosis and optionally a voice recognition module for translating the intercepted dictation into a text format. Optionally, to initiate dictation, the user selects the section in the report template to which she want to relate. Optionally, the microphone is used for intercepting the dictation and the report generation module 202 is used for storing the intercepted dictation in association with the report, for example with one or more related sections of the report.

As shown at 106, the report may be adjusted, optionally dynamically, according to inputs of the user. The report may be adjusted during the completing thereof by the user, for example during the dictation of the diagnosis. In such a manner, the report may be dynamically adjusted according to a pathology that has been diagnosed by the user, a suggested treatment, a transferal to receive a treatment, and a transferal for acquiring additional medical imaging study. Optionally, the report and the imaging study and/or the medical records of the patient are presented to the user on the same client terminal, optionally substantially simultaneously.

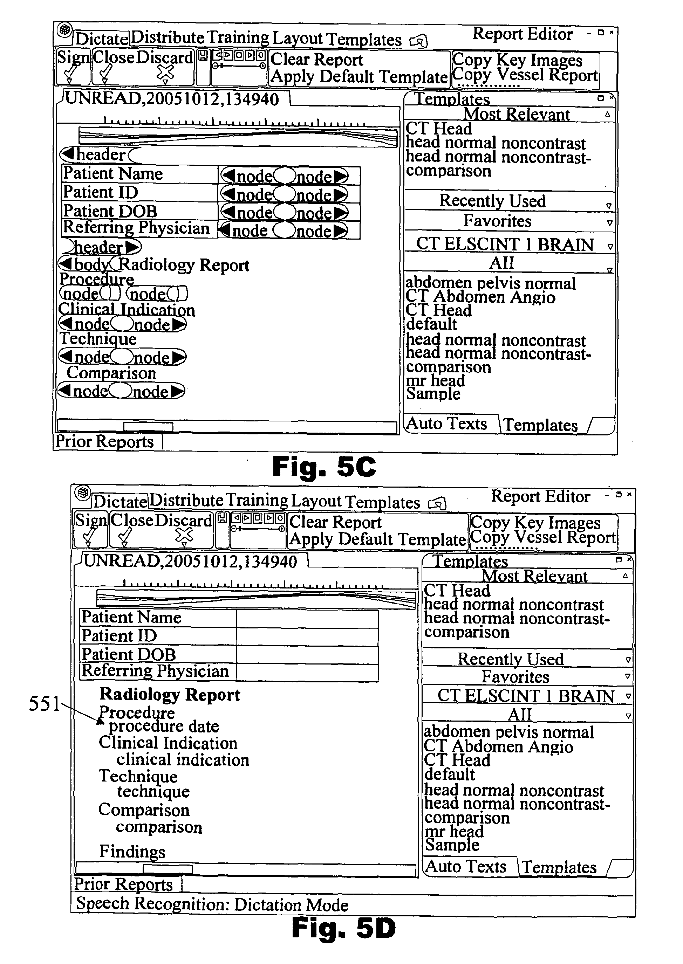

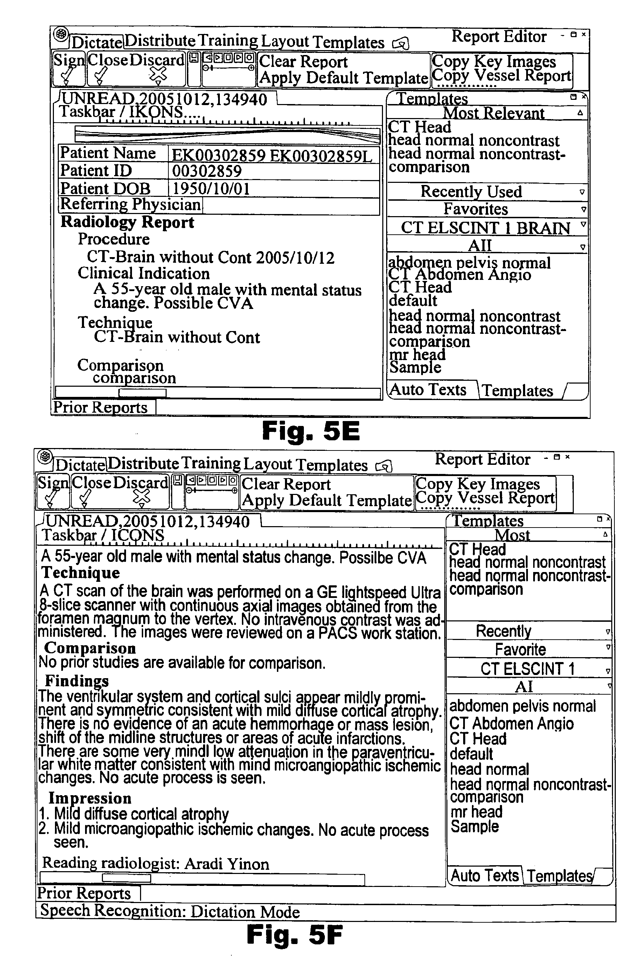

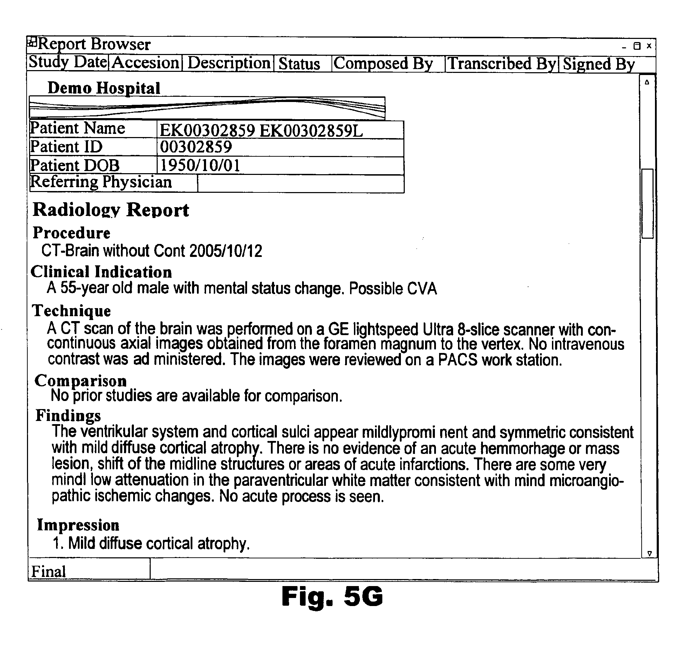

Reference is now also made to FIGs. 5C-5G, which are a set of screenshots that illustrates the process of a report generating, for example according to the method depicted in FIG. 1 and described above. FIG. 5C is a screen shot of an empty report template which was selected for a particular imaging study, for example according to the descriptive data which is related to the received imaging study and/or initial inputs of the user. The fields which are designed to filled automatically are encircled with a contour. It should be noted that these contours are illustrative and does not presented to the user. FIG. 5D is an exemplary screenshot of a screen which is presented to the user after the report is selected according to the received imaging study and/or initial inputs of the user. As shown at 551, some of the fields are automatically filled in with relevant placeholder text. FIG 5E depicts the selected and/or generated report of FIGs. 5A and 5B, with automatically filled in data. FIG. 5F is a screenshot of a screen of the automatically filled in report of FIG. 5E after the user added his input, by typing, dictating, and/or using various macro features. FIG. 5G is a blowup of the filled section. As depicted, the report now contains the technique used and the findings which are based, *inter alia,* on the imaging study. FIG. 5E is a screenshot of a screen of an exemplary final report as outputted, as shown at 106, presented and/or distributed.

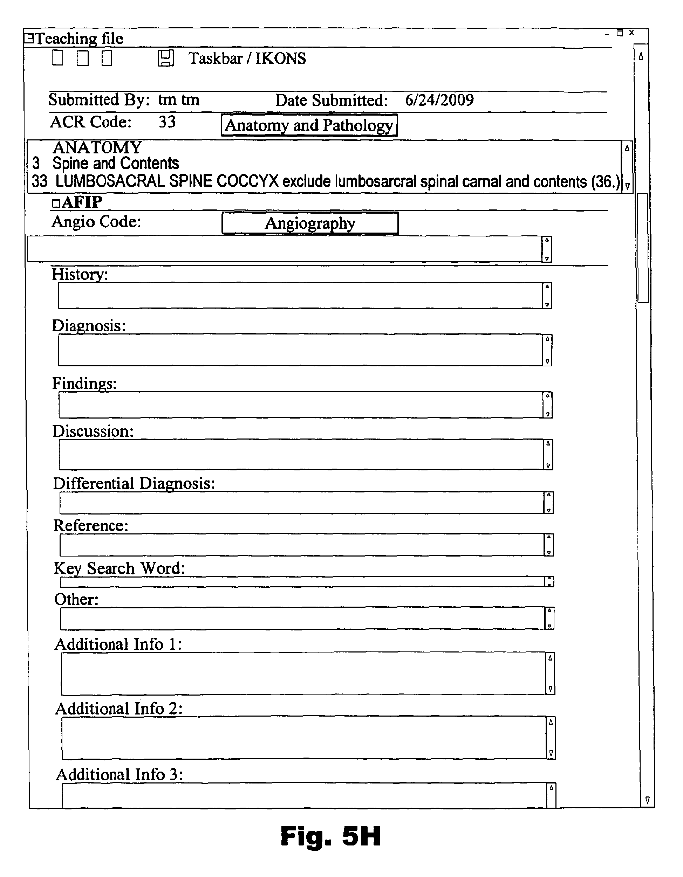

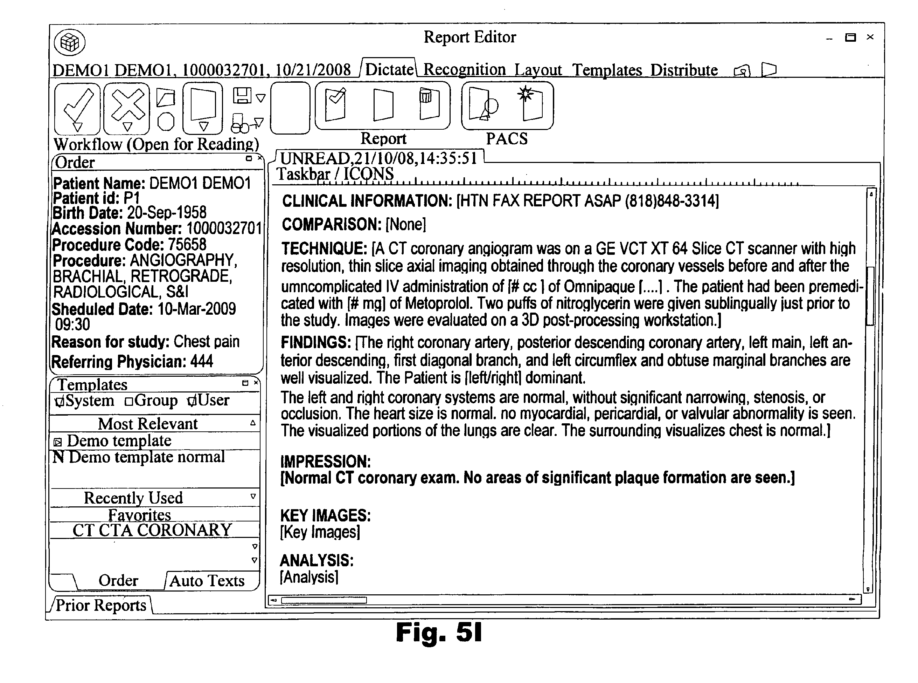

Additionally or alternatively, a teaching file is loaded, optionally similarly to the process of a report generating that is described above and depicted in FIG. 1. As commonly known, a teaching file is a feature that enables radiologists to keep cases for teaching purposes, for example. A teaching file usually includes an imaging study and a text segment describing the findings, the history, medical information, such as demographics, a diagnosis, optionally including differential diagnosis and the like. Optionally, a teaching file report is automatically filed, using the process of a report generating which is described in FIG. 1. The teaching file report is designed to facilitate the creation of a teaching file, as known in the art. For example, a report as depicted in FIG. 5H is filed to create a report as presented in FIG. 5I. After the automatic or semi automatic filing process data fields, such as medical history, diagnosis, finding, a discussion, and a differential diagnosis from the report may be available to fill the teaching file report. The fields may be copied, optionally automatically, to the teaching file record which is hosted locally and/or in a teaching repository. Optionally, additional information is obtained automatically from the imaging study, for example technical information, including the type of study, the use of contrast media, and the like. Optionally, additional information, such as current medical status, reasoning for requesting the imaging study and the like may be automatically obtained from the imaging study order form. Optionally, one or more designated rubrics are added for allowing the physician to update data pertaining to teaching files during the reporting process. Optionally, the teaching file report is presented to the physician before the transmission thereof to the teaching database. It should be noted that by adding rubrics, which are related to the teaching file report, and/or steps, such as sending a teaching file report and/or operational steps such as ratifying the teaching file report before the transmission thereof to a teaching database, the compliance of the physician to create a teaching file is increased. In such an embodiment, the teaching file is created as a by product of the reporting process and therefore the physician does not have to perform designated operations in order to create the teaching file or has to perform less operations than usually required. Mapping of the various fields of the report, the imaging study order, and/or the imaging study into the fields of the teaching file report allows filling a large portion of the teaching file report and may leave only a minor portion to be filled manually by the physician. Fine tuning of the teaching file report may also be performed by the physician and/or an operator of the teaching database.

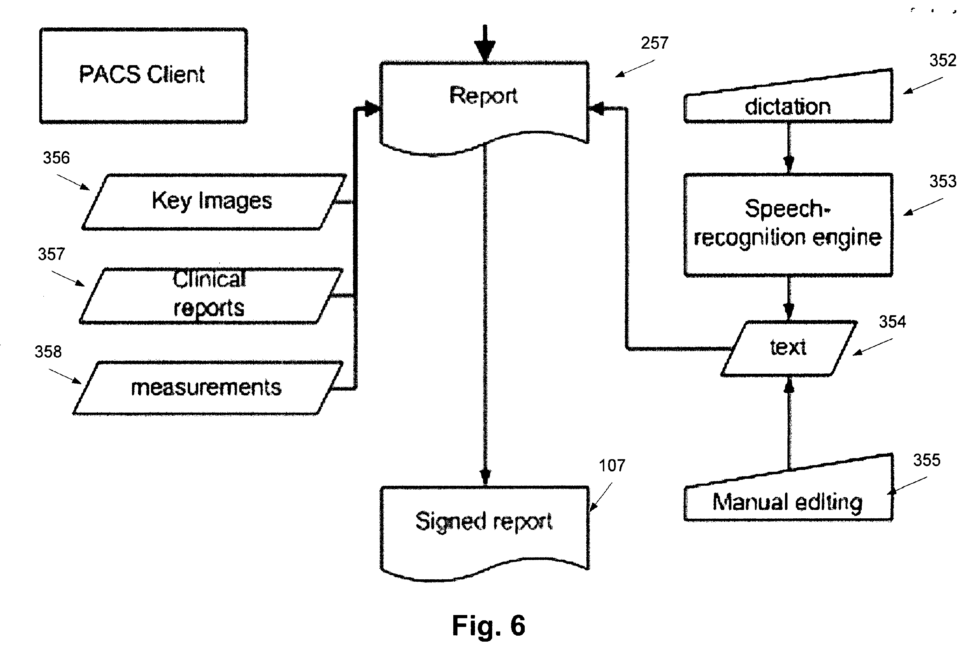

Reference is now made to FIG. 6, which is a flowchart of a diagnosis process, according to some embodiments of the present invention. As depicted in FIG. 6, the report 351 may be adjusted and/or filled in according to data that gathered from a plurality of different sources. The sources may be a text which is a translation of the dictation of the user, for example as described above and shown at 352-354, a text that has been added manually by the user, as shown at 355, and one or more medical data elements which are received and/or selected by the user. For example, the user may add clinical reports 356, such as structured reports generated by modalities such as DICOM SR, vessel analysis and calcium scoring reports, select key images from the medical studies 357, and/or add measurements and image annotations which are related to her diagnosis, as shown at 358. Optionally, the report generation module 202 adjusts the report to fit the one or more medical data elements and/or selections. For example, the report may present rubrics which are related to the selected images or to the received clinical reports.

Additionally or alternatively, the report generation module 202 adds the received medical imaging study or images therefrom to the report according to the inputs of the user. Optionally, the segments, which are added to the report, define anatomic sites, each referred to in the inputs. In such a manner, the report may provide a visual reference to the diagnosis of the user. Optionally, the MMI 203 allows the user to mark anatomical sites on the segments of the medical imaging study which have been added to the report. In such a manner, the user may refer the reader to specific areas of interest. Optionally, the report generation module 202 includes a voice recognition module and/or a text analysis module which are used for identifying references to anatomical sites in the inputs of the user. In such an embodiment, the report generation module 202 may select segments of the imagining study according to the identified anatomical sites and add them to the report in association with a respective section in the diagnosis. Alternatively or additionally the report generation module 202 may mark segments of the imagining study according to the identified anatomical sites and associate them respective sections of the report.

Optionally, the report generation module 202 is connected to a computer aid diagnosis (CAD) system. In such an embodiment, the CAD may receive and process the one or more diagnosed medical imaging studies and output an automated analysis accordingly. Optionally, the automated analysis is added to the report and/or used for automatically update pf the report.

According to some embodiments of the present invention, the imaging study is presented to the user according to a protocol which have been selected according to the modality and/or the anatomical site which is related thereto. Optionally, the imaging study comprises a set of views, such as posterior, anterior, lateral, superior and/or interior views. In such an embodiment, the views may be presented sequentially. Each presented view allows the user to relate thereto and to determine when to present the following view. Optionally, the views are added in a sequential manner to the report, optionally each with an association to the related diagnosis which has been provided by the user. In such a manner, the report that is outputted in the end of the medical reporting session, for example as shown at 107, may be generated in a manner that each diagnosis is presented with the view on which it is based. Optionally, the sequence is dynamically adjusted according to the inputs of the user. In such a manner, the order in which the views are presented to the user is adjusted according to her inputs.

Now, as shown at 107, the report is outputted. Optionally, as shown at 107, the report is signed, for instance with a digital signature, and forwarded, for example using an output unit 212, to a referring agent, for example to a referring physician and/or to a reporting database that is intended for use as a scalable shared database. As described above, the generated report includes rich content such as text, measurements, and images, for example as depicted in FIG. 5B. Optionally, the reports further comprise rich data from other sources, such as hyperlinks, tables, and graphs which are based on a combination of inputs from the user and/or the received medical imaging studies and/or medical records.

Reference is now made to FIG. 7, which is a flowchart of a method for preparing a radiology report pertaining to a patient, according to some embodiments of the present invention. Blocks 101-107 are as depicted in FIG. 1 however FIG. 7 further depicts block 108-111.

As shown at 108, the report template may be selected according to selections of the user. In such an embodiment, the user's diagnosis input, for example by using the MMI 203, one or more selections or definitions, which may be referred to herein as diagnosis indicators, that indicate which diagnosis or prognosis she is about to perform and/or which report template she rather use. Optionally, the report template may be selected according to the user profile of the user. In such an embodiment, the client terminal 100 and/or a central server is used to maintain a user profile that documents the user's selections and/or preferences. Optionally, the user profile stores the selections which have been made by the user in previous diagnosis sessions and the report template is selected according to the previously selected report templates. For example, if the user selected template A for diagnosis of a medical imaging study of anatomic site X from modality Y, the same report template may be automatically selected by the report generation module 202 when the user diagnoses other medical imaging studies of anatomic site X from modality Y. Optionally, the client terminal displays a GUI that is adjusted for allowing the user to adjust his preference and/or to rank the report templates and/or sections thereof. In such am embodiment, the user may determine which report templates and/or report template sections are loaded for her use during medical reporting sessions.

Additionally or alternatively the report template may be selected according to the reports which have been previously used with the probed patient.

As shown at 110, the coherency of the data that is inputted by the user, for example by means of dictating and/or typing, may be verified with respect to the one or more acquired medical imaging studies and/or medical records and/or with respect to other inputs which have been inputted by the user. For example, as described above, the report template may be selected and/or adjusted according to the anatomic site which is depicted in the medical imaging study. If this anatomic site is different from the anatomic site that is referred to in the data that is inputted by the user, the coherency check fails. Optionally, if the coherency check fails, the MMI 203 alarms the user. Optionally, if the coherency check fails, the report generation module 202 adds an incoherency notice to the report.

An example for an incoherency that may be detected in such a coherency check is the detection of a reference to the left femur of the patient in the user's diagnosis inputs in a report that is based on a medical imaging study that refers to the right femur of the patient

As shown at 110, the coherency of the conclusions of the report, for may be verified with respect to the user's diagnosis input and/or, the acquired one or more medical imaging studies and/or medical records. In a common medical reporting session workflow, the user summarizes her diagnosis in a conclusions section. Usually, the conclusions section refers to anatomical sites and pathologies which have been referred to in the body of the report and depicted in the medical imaging study. Optionally, the report generation module 202 verify that the anatomical sites and/or pathologies which are referred to in the conclusions section are coherent with the anatomical sites and/or pathologies which are referred to in the body of the report and/or with the anatomical sites and/or pathologies which have been identified in the one or more acquired medical imaging studies and/or medical records.

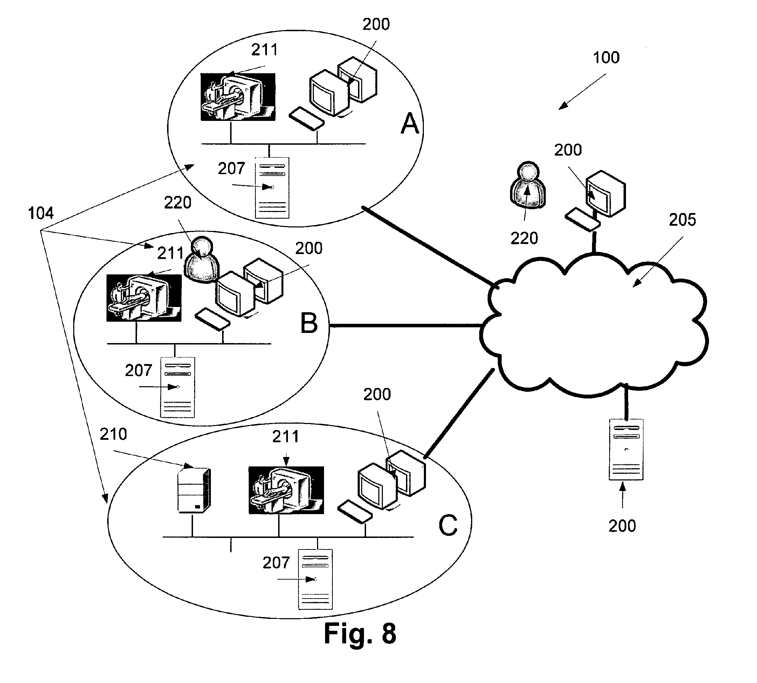

Reference is now made to FIG. 8, which is a schematic illustration of a platform 300 for managing medical data records which are stored in multiple local medical imaging systems 301, for example as described in Patent Application No. 61/071,709 and Patent application No. 61/071,708 co-filed on May 14, 2008, which are incorporated herein by reference. Numerals 200, 210, 211, and 205 are as depicted in FIG. 2 however the system 300 that is depicts in FIG. 8 further comprises a plurality of integration devices 207 that allows the medical imaging study and medical record systems of different types to communicate among each other, as fully described in Patent Applications No. 61/071,709 and No. 61/071,708, which are incorporated herein by reference.

In some embodiments of the present invention one or more of the local medical imaging systems 301 are PACS networks. In such embodiments, each local medical imaging system 301 consists of a central server, such as a DICOM server, that stores a plurality of medical imaging studies, such as outcomes of medical imaging, for example medical imaging studies, and connected to one or more client terminals 200, optionally via a LAN and/or a wide area network (WAN).

Each local medical imaging system 301, which is optionally an independent PACS, may include web-based interfaces to utilize the Internet or any other computer network, as a device of communication, for example via a virtual private network (VPN) or a secure sockets layer (SSL) connection. In such an embodiment, each local medical imaging system 301 may include a number of client terminals 200 which are located in a remote location. For example, a PACS of a hospital may allow users, such as the hospital radiologists, to access DICOM objects which are stored in a local DICOM server from a client terminal 200, such as a personal computer, that is located in his office, his home, and/or in another hospital.

The platform 300 allows a user, which is connected to a certain client terminal 200 of one of the local medical imaging systems 301, to acquire medical imaging studies and medical records, which is stored in storage devices 210, 211 and/or other client terminals which are associated with other local medical imaging systems 301, for example as fully described in US. Patent Applications No. 61/071,709 and No. 61/071,708.

In some embodiments of the present invention, the platform 300 is connected to a central server 302 that gathers statistical information about the medical reporting sessions which have been performed by the client terminals 200. The statistical information may be based on the reports that users selected and/or on questions and answers (Q&A) sessions which have been established by the central server 302 and/or the report generation module 202 after the medical reporting session. Such Q&A session may be used for estimating the suitability of the adjusted report to the user needs. In such an embodiment, a Q&A session may be held after a respective medical reporting session. The outcome of the Q&A sessions may be stored and analyzed in the central server 302.

In such a manner, the central server 302 may score the suitability of some or all of the report templates in the report template dictionary to one or more characteristics of a medical imaging study, for example to characteristics such as a modality, an anatomic site, a pathology, and any combination thereof. Such a scoring may be used as a basis for matching a report by the report generation module 202 of the client terminal 200, as further described above.

It is expected that during the life of a patent maturing from this application many relevant methods and systems will be developed and the scope of the term studies, medical records, and reports is intended to include all such new technologies *a priori.*

As used herein the term "about" refers to ± 10 %.

The terms "comprises", "comprising", "includes", "including", "having" and their conjugates mean "including but not limited to".

The term "consisting of means "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. A method for supporting a preparation of medical report for a patient, comprising:
acquiring at least one of a medical imaging study and a medical record related to the patient;
automatically matching a report template to said at least one of said medical imaging study and said medical record according to at least one characteristic thereof;
presenting said matched report template to allow a user to provide a diagnosis of said at least one of said medical imaging study and said medical record; and
embedding said diagnosis in said matched report template.

2. The method of claim 1, said automatically matching comprises automatically matching said report template according to a combination of said medical imaging study and said medical record.

3. The method according to any of claims 1-2, wherein said allowing comprises dynamically adjusting said report according to said diagnosis.

4. The method according to any of claims 1-3, wherein said matching comprises analyzing said at least one medical imaging study to identify an anatomic site in the body of said patient and matching said report according to said anatomic site.

5. The method according to any of claims 1-4, wherein said report template is matched from a plurality of report templates, said acquiring comprising acquiring statistical data related to at least one previously selected report, said matching being performed according to said statistical data.

6. The method according to any of claims 1-5, further comprising documenting said matching, wherein said documenting allows adjusting at least one prospective report, said at least one prospective report being filled in by said user.

7. The method according to any of claims 1-6, wherein said embedding comprises automatically identifying at least one of a miscorrelation between said at least one of said medical imaging study and said medical record an anomaly in said at least one of said medical imaging study and said medical record, further comprising presenting at least onf of said miscorrelation and said anomaly to said user.

8. The method according to any of claims 1-7, wherein said acquiring extracting billing information from said at least one of said medical imaging study and said medical record, said matching being performed according to said billing information.

9. The method according to any of claims 1-8, further comprising confirming a coherency between said diagnosis and data extracted from said at least one of said medical imaging study and said medical record.

10. The method according to any of claims 1-9, further comprising using a computer aid diagnosis (CAD) for processing said at least one medical imaging study and updating said report according to said processing.

11. The method according to any of claims 1-10, wherein said matching comprises matching a radiology analysis standard according to said at least one of said medical imaging study and said medical record and analyzing said diagnosis to verify a compliance of said user with said matched radiology analysis standard.

12. The method according to any of claims 1-11, further comprising segmenting at least one anatomic site in said at least one medical imaging study and adjusting said report according to said segmenting.

13. The method according to any of claims 1-12, further comprising identifying a match between at least one of said diagnosis and said at least one medical imaging study and at least one of a plurality of exemplary medical imaging study reports, and adding said at least one exemplary medical imaging study report to said report.

14. A client terminal for preparing a medical report, comprising:
an acquiring module configured for acquiring at least one of a medical record and a medical imaging study related to a patient;
a report generation module configured for generating a report by matching a report template of a plurality of report templates to said at least one of said medical record and said medical imaging study; and
a man machine interface (MMI) configured for allowing a user to provide a diagnosis about said at least one of said medical record and said medical imaging study;
wherein said diagnosis is embedded into said report template to form the medical report.

15. The client terminal of claim 14, wherein said report generation module is configured for at least one of dynamically adjusting said report according to said diagnosis and generating a teaching file according to said diagnosis.
